# EUROPEAN PATENT APPLICATION

(11) **EP 3 756 542 A1**
(43) Date of publication of application: **30.12.2020**
(21) Application number: 19182273.3
(22) Date of filing: 25.06.2019
(51) Int. Cl.: A61B 5/0484

(54) **APPARATUS FOR PATIENT SEDATION MONITORING**

(71) Applicant: Koninklijke Philips N.V., 5656 AG Eindhoven (NL)
(72) Inventor: SISODIA, Rajendra Singh, 5656 AE Eindhoven (NL); JOHNSON, Mark Thomas, 5656 AE Eindhoven (NL); BUSSA, Nagaraju, 5656 AE Eindhoven (NL); VUPPALA, Sunil Kumar, 5656 AE Eindhoven (NL); HELLE, Michael Günter, 5656 AE Eindhoven (NL); WEISS, Steffen, 5656 AE Eindhoven (NL)
(74) Representative: de Haan, Poul Erik

(57) **Abstract**

The present invention relates to an apparatus (10) for patient sedation monitoring. The apparatus comprises a plurality of patient stimulation devices (20), at least one sensor device (30), and a processing unit (40). Each patient stimulation device of the plurality of patient stimulation devices is configured to provide a mode of stimulation. The modes of stimulation for the plurality of patient stimulation devices are different to each other. The plurality of patient stimulation devices is configured to provide a multi-modal stimuli to a sedated patient. The at least one sensor device is configured to acquire at least one patient response to the multi-modal stimuli. The processing unit is configured to determine a sedation state of the sedated patient comprising utilization of the at least one patient response to the multi-modal stimuli.

## Description

### FIELD OF THE INVENTION

The present invention relates to an apparatus for patient sedation monitoring, an image acquisition system, a method for patient sedation monitoring, a method of image acquisition, as well as to a computer program element and a computer readable medium.

### BACKGROUND OF THE INVENTION

When patients who are sedated and are undergoing medical scans, for example utilizing a Magnetic Resonance MR imaging unit, a radiography X-ray imaging unit, a computer tomography imaging CTI X-ray unit, a positron emission tomography PET unit, are required to have their sedation state frequently evaluated. However, the identification of the objective sedation status is one of the key problems when imaging the patient in order to select the right protocol with an adapted timing sequence and also to identify how the sedation status is changing to decide on next steps. It is particularly challenging to assess the sedation state in the noisy environment of a scanner system or imaging unit.

There are many established protocols for evaluating sedated patients and to assess their level of sedation. Almost all of them evaluate on a scale from a lowest to highest scale, where for example the highest scale is based on the absence of any response from the patient to a stimulus that would have been expected to result in a highly responsive action. The assessment of the response to such stimuli is descriptive with human observable parameters. The stimuli described in such assessment are not standard and suffer from a single category of stimulus. Since each person has different response to a type of stimulus, they do not inspire confidence. As such the presence of expert anesthetists/doctors is always required near to the subject.

CN104921723A describes a conscious state detection method based on a multi-mode brain-computer interface. The conscious state detection method includes the steps that two photos are displayed on a graphical user interface, and a user wanting to select one photo can keep an eye on the corresponding photo and silently count the times of flicking of a photo frame; SSVEP (steady state visual evoked potential) is generated by means of flicking of the corresponding photo, and P300 electric potential is generated by means of flicking of the photo frame; a scalp electroencephalogram signal is acquired via a head-wear electroencephalogram acquiring hat and is recorded by a portable amplifier; the scalp electroencephalogram signal is duplicated in two copies which enter into P300 electric potential detection and SSVEP detection respectively; normal conscious state of the user is assessed and determined according to detection results. It is described that the conscious state detection method based on the multi-mode brain-computer interface has the advantages that whether the user can recognize a specified object (a photo of the own face or a photo of another's face) or not is determined by means of detecting P300 and SSVEP of the user, and whether the user is conscious or not can be recognized effectively.

However, there is a need to improve how the sedation state of a patient can be monitored.

### SUMMARY OF THE INVENTION

It would be advantageous to have improved means of monitoring the sedation state of a patient. The object of the present invention is solved with the subject matter of the independent claims, wherein further embodiments are incorporated in the dependent claims. It should be noted that the following described aspects and examples of the invention apply also to the apparatus for patient sedation monitoring, image acquisition systems, methods for patient sedation monitoring, and the method of image acquisition, as well as to the computer program element and a computer readable medium.

In a first aspect, there is provided an apparatus for patient sedation monitoring, comprising:
- a plurality of patient stimulation devices;
- at least one sensor device; and
- a processing unit.

Each patient stimulation device of the plurality of patient stimulation devices is configured to provide a mode of stimulation, and the modes of stimulation for the plurality of patient stimulation devices are different to each other. The plurality of patient stimulation devices is configured to provide a multi-modal stimuli to a sedated patient. The at least one sensor device is configured to acquire at least one patient response to the multi-modal stimuli. The processing unit is configured to determine a sedation state of the sedated patient comprising utilization of the at least one patient response to the multi-modal stimuli.

In other words, a sedation level determination apparatus is provided that can determine the sedation level of a patient for utilization in for example a medical scan procedure.

In this manner, a multi-modal stimuli generation apparatus combines multiple sensory modality to increase the chance of a response for a given sedated state.

In other words, a reliable assessment of sedation is enabled through a patient being provided with a number of stimuli that invokes at least one observable response, which can then be objectively assessed as per a defined procedure/standard sedation assessment scale in order to provide an accurate and repeatable determination of their sedation state.

In an example, the plurality of patient stimulation devices are configured to operate in a frequency range of 0.1Hz to 10Hz.

In an example, the plurality of patient stimulation devices are configured to provide the modes of stimulation simultaneously to provide the multi-modal stimuli to the sedated patient.

Thus, the multi-modal stimuli are provided at the same time, congruently, and it has been found that this elicits an improved ability to determine the sedation state of the patient from how the respond to such congruent multimode stimuli.

In an example, the plurality of patient stimulation devices are configured to provide the modes of stimulation congruently to provide the multi-modal stimuli to the sedated patient.

In this way, the different modes of stimuli can be applied in a similar manner, for example having similar or the same frequency waveforms, amplitude waveforms. In this way, the patient is stimulated such that the sensory organs are lead to believe that there is a single source of stimulation, albeit from different sources that can target different sensory organs, but where the response to this stimulation is increased.

By using congruent multi-modal stimulation and monitoring the patient, better determination of the sedation state of the patient has been found to be enabled over that for a single stimuli technique.

To put this another way, a congruent, repeatable and objective multi-modal stimuli delivery apparatus is provided and the response from the sedated patient is elicited, and the response or lack of response based on such stimuli provides a more reliable sedation state assessment than currently available.

In an example, the plurality of patient stimulation devices are configured to provide stimuli targeted at a plurality of sensory organs of the sedated patient.

In this manner, by targeting different sensory organs, if one responsive mechanism of a particular patient is not receptive, by targeting other response mechanisms and overall "picture" of the sedation state can be determined from the responses from a number of different sensory organs. In this way, a more accurate and consistent sedation state of the patient can be determined.

In an example, a first patient stimulation device is configured to target a first sensory organ and a second patient stimulation device is configured to target a second sensory organ.

In an example, the processing unit is configured to select the plurality of patient stimulation devices from a larger number of potential patient stimulation devices, the selection comprising utilization of sensory information relating to the sedated patient.

In other words, if the patient is determined to be hard of hearing and/or visually impaired, then it can be determined that audible stimuli and/or visual stimuli will not be selected. Additionally, if for example it is determined that the patient is cognitively impaired, then it can be determined that the asking of questions to the patient will not be used.

Thus, the combined multi-modality is personalized for the patient's specific sensory model.

In an example, the at least one patient response comprises a plurality of patient responses, wherein the plurality of patient response are different to each other.

Thus, the multi-modal stimulation results in an overall response that is greater than a response for a single form of stimulation, but that overall increased response can manifest itself in individual responses that are individually greater than they would have been if there was only that form of stimulation.

In a second aspect, there is provided an image acquisition system, comprising:
- an image acquisition unit; and
- an apparatus for patient sedation monitoring according to the first aspect.

The image acquisition unit is configured to acquire image data of the sedated patient. The processing unit is configured to determine at least one scan protocol and/or terminate a scan protocol for the image acquisition unit for the acquisition of the image data comprising utilization of the determined sedation state of the patient.

Thus, the probability of a correct assessment of the sedation state of the patient is increased, and a more efficient scan procedure can be achieved.

In this manner, multimodal stimuli, which can be congruent, and can be adapted to the modality, patient and environment are used to accurately determine the sedation state of the patient. The automated delivery of multiple stimulus is combined with their assessment in a congruent manner that is both re-producible, objective and easily deployable in autonomous imaging system, and that enables the imaging system to take into account the sedation state.

Also, for example, if sedation is beginning to wear off and it will take time before the administration of new sedatives will provide the correct level of sedation, the scan can be halted to ensure that the patient does not for example become aware of the scan and start to panic.

In a third aspect, there is provided a method for patient sedation monitoring, comprising:
- providing by a plurality of patient stimulation devices a multi-modal stimuli to a sedated patient, wherein each patient stimulation device of the plurality of patient stimulation devices is configured to provide a mode of stimulation, and wherein the modes of stimulation for the plurality of patient stimulation devices are different to each other
- acquiring by at least one sensor device at least one patient response to the multi-modal stimuli; and
- determining a sedation state of the sedated patient comprising utilization of the at least one patient response to the multi-modal stimuli.

In an example, step d) is carried out by a processing unit, and wherein the method comprises step e) outputting by an output unit the sedation state of the sedated patient.

In an example, the method comprises step a) selecting the plurality of patient stimulation devices from a larger number of potential patient stimulation devices, the selection comprising utilizing sensory information relating to the sedated patient.

In an example, in step c) the at least one patient response comprises a plurality of patient responses, wherein the plurality of patient response are different to each other.

In a fourth aspect, there is provided a method of image acquisition comprising:
a1) determining a sedation state of a sedated patient in an image acquisition unit according to the method of the third aspect;
b1) determining by a processing unit at least one scan protocol for the image acquisition unit for the acquisition of image data comprising utilization of the determined sedation state of the patient.

According to another aspect, there is provided a computer program element controlling one or more of the apparatuses as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an apparatus for patient sedation monitoring;
Fig. 2 shows a schematic set up of an example of an image acquisition system that utilizes an apparatus for patient sedation monitoring;
Fig. 3 shows an example of a method for patient sedation monitoring;
Fig. 4 shows an example of a method of image acquisition;
Fig. 5 shows a schematic example of a multi-sensory system;
Fig. 6 shows a detailed workflow of an apparatus for patient sedation monitoring;
Fig. 7 shows a set of three waveforms, representing an audio, temperature and head movement stimuli.

### DETAILED DESCRIPTION OF EMBODIMENTS

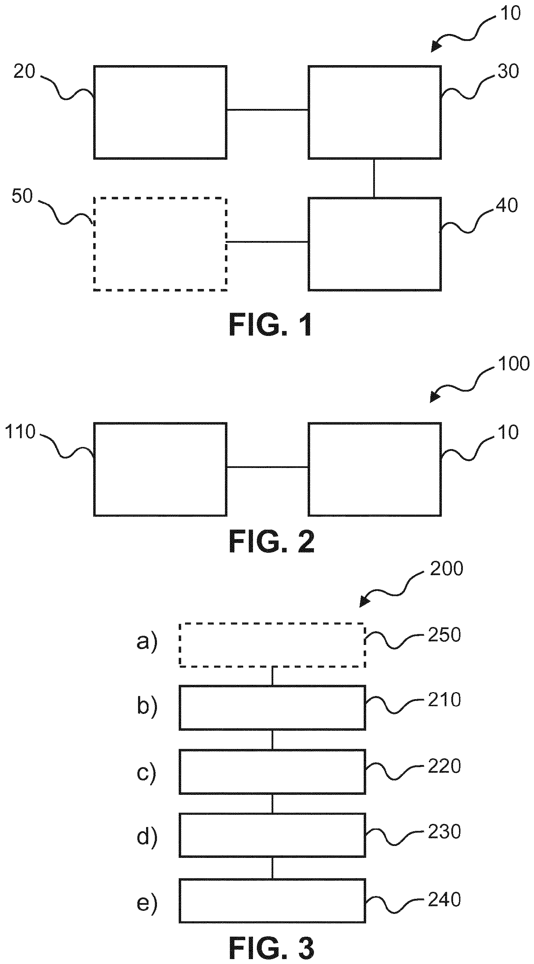

Fig. 1 shows an example of an apparatus 10 for patient sedation monitoring, where essential features are shown in solid lines and optional features are shown in dashed lines. The apparatus 10 comprises a plurality of patient stimulation devices 20, at least one sensor device 30, and a processing unit 40. Each patient stimulation device of the plurality of patient stimulation devices is configured to provide a mode of stimulation. The modes of stimulation for the plurality of patient stimulation devices are different to each other. The plurality of patient stimulation devices is configured to provide a multi-modal stimuli to a sedated patient. The at least one sensor device is configured to acquire at least one patient response to the multi-modal stimuli. The processing unit is configured to determine a sedation state of the sedated patient comprising utilization of the at least one patient response to the multi-modal stimuli.

In an example, the apparatus comprises an output unit (50) configured to output the sedation state of the sedated patient.

In an example, a generating unit is configured to generate the multi-modal stimuli.

In an example, an adaptation unit is configured to adapt the generated stimuli to the patient when sedated.

In an example, a delivery unit is configured to deliver the multi-modal stimuli to the patient.

In an example a multi-modal stimuli response assessment unit is configured to determine the responses of the patient to the multi-modal stimuli.

In an example, output unit can be used to adapt a medical scan procedure based on the responses.

According to an example, the plurality of patient stimulation devices are configured to operate in a frequency range of 0.1Hz to 10Hz.

In an example, the plurality of patient stimulation devices are configured to operate in a modulation frequency range of 0.1Hz to 10Hz

In an example, the plurality of patient stimulation devices operate in a frequency range of 1/9Hz to 3Hz.

In an example, the plurality of patient stimulation devices operate in a frequency range of 0.2Hz to 2Hz.

In an example, the plurality of patient stimulation devices are configured to stimuli comprising: mechanical haptic stimuli; TENS stimuli; visual stimuli; audio stimuli; nerve and/or muscle stimuli via a Magnetic Resonance Imaging unit; questions posed; taste stimuli; fragrance stimuli; balance related stimuli (rocking of the head for example).

In an example, the at least one sensor device comprises: a camera, an EMG sensor, a movement sensor, a tilt sensor, an accelerometer, a microphone, and for the nerve and/or muscle stimuli the at least one sensor device can be the Magnetic Resonance Imaging unit itself.

According to an example, the plurality of patient stimulation devices are configured to provide the modes of stimulation simultaneously to provide the multi-modal stimuli to the sedated patient.

According to an example, the plurality of patient stimulation devices are configured to provide the modes of stimulation congruently to provide the multi-modal stimuli to the sedated patient.

According to an example, the plurality of patient stimulation devices are configured to provide stimuli targeted at a plurality of sensory organs of the sedated patient.

According to an example, a first patient stimulation device is configured to target a first sensory organ and a second patient stimulation device is configured to target a second sensory organ.

In an example, each patient stimulation device is configured to target a different sensory organ.

According to an example, the processing unit is configured to select the plurality of patient stimulation devices from a larger number of potential patient stimulation devices. The selection comprises utilization of sensory information relating to the sedated patient.

According to an example, the at least one patient response comprises a plurality of patient responses, wherein the plurality of patient response are different to each other.

In an example, the processing unit is configured to determine a specific sedation state indicator for each of the specific patient response, and wherein determination of the sedation state comprises utilization of the plurality of specific sedation state indicators. In other words, the assessment of sedation state can linked with the type(s) of stimulus.

Fig. 2 shows an example of an image acquisition system 100. The system comprises an image acquisition unit 110, and an apparatus 10 for patient sedation monitoring as described with respect to Fig. 1. The image acquisition unit is configured to acquire image data of the sedated patient. The processing unit of the apparatus 10 is configured to determine at least one scan protocol for the image acquisition unit for the acquisition of the image data comprising utilization of the determined sedation state of the patient. Additionally or alternatively the processing unit of the apparatus 10 is configured to terminate a scan protocol for the image acquisition unit for the acquisition of the image data comprising utilization of the determined sedation state of the patient.

In an example, determination of the scan protocol comprise halting a scan if it is determined that the sedation state of the patient has become less that that required for the scan.

In an example, determination of the scan protocol comprise starting a scan if it is determined that the sedation state of the patient is commensurate with that required for the scan.

In an example, determination of the scan protocol comprise starting a particularly loud part of a scan if it is determined that the sedation state of the patient is commensurate with that required for that part of the overall scan protocol.

Fig. 3 shows a method 200 for patient sedation monitoring in its basic steps where essential steps are shown in bold, and where optional steps are shown in dashed. The method 200 comprises:
- in a providing step 210, also referred to as step b), providing by a plurality of patient stimulation devices a multi-modal stimuli to a sedated patient, wherein each patient stimulation device of the plurality of patient stimulation devices is configured to provide a mode of stimulation, and wherein the modes of stimulation for the plurality of patient stimulation devices are different to each other
- in an acquiring step 220, also referred to as step c), acquiring by at least one sensor device at least one patient response to the multi-modal stimuli; and
- in a determining step 230, also referred to as step d), determining a sedation state of the sedated patient comprising utilization of the at least one patient response to the multi-modal stimuli.

In an example, step d) is carried out manually.

According to an example, step d) is carried out by a processing unit, and wherein the method comprises step e) outputting 240 by an output unit the sedation state of the sedated patient.

In an example, the plurality of patient stimulation devices provide the modes of stimulation simultaneously to provide the multi-modal stimuli to the sedated patient. In an example, the plurality of patient stimulation devices provide the modes of stimulation congruently to provide the multi-modal stimuli to the sedated patient.

In an example, the plurality of patient stimulation devices provide stimuli targeting at a plurality of sensory organs of the sedated patient.

In an example, a first patient stimulation device targets a first sensory organ and a second patient stimulation device targets a second sensory organ.

In an example, each patient stimulation device targets a different sensory organ.

According to an example, the method comprises step a) selecting 250 the plurality of patient stimulation devices from a larger number of potential patient stimulation devices, the selection comprising utilizing sensory information relating to the sedated patient.

In an example, selecting the plurality of patient stimulation devices from a larger number of potential patient stimulation devices is carried out manually.

In an example, selecting the plurality of patient stimulation devices from a larger number of potential patient stimulation devices is carried out by the processing unit.

According to an example, in step c) the at least one patient response comprises a plurality of patient responses, wherein the plurality of patient response are different to each other.

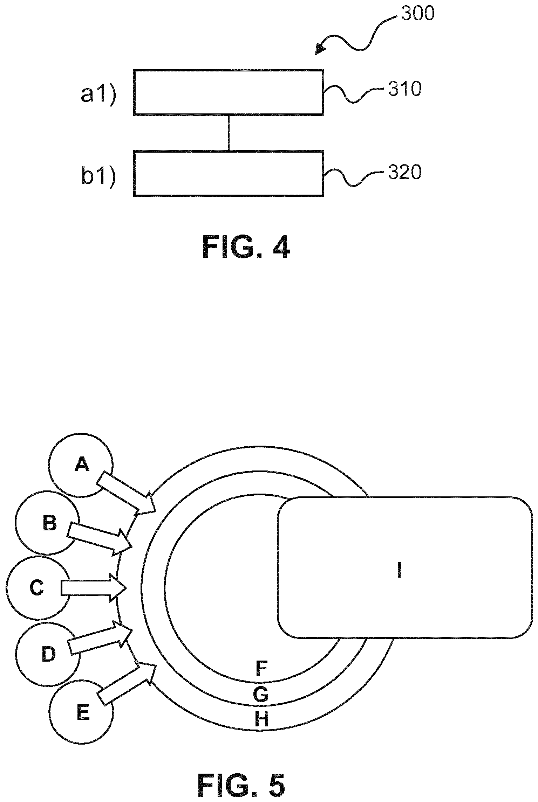

Fig. 4 shows a method 300 of image acquisition in its basic steps. The method 300 comprises:
- in a determining step 310, also referred to as step a1), determining a sedation state of a sedated patient in an image acquisition unit according to the method 200 as described with respect to Fig. 3; and
- in a determining step 320, also referred to as step b1), determining by a processing unit at least one scan protocol and/or terminating a scan protocol for the image acquisition unit for the acquisition of image data comprising utilization of the determined sedation state of the patient.

In an example, the processing unit that determines the at least one scan protocol and/or that terminates a scan protocol is the processing unit that determines the sedation state.

In an example, the processing unit that determines the at least one scan protocol and/or that terminates a scan protocol is a processing unit of the image acquisition unit.

The apparatus for patient sedation monitoring, image acquisition system, method for patient sedation monitoring, and the method of image acquisition are now described in more detail with respect to specific embodiments, where reference is made to Figs. 5-7.

In seeking to determine an improved technique to determine the sedation state of a patient undergoing a medical scan, the paper by E. Garcia-Ceja et al: "Mental health monitoring with multimodal sensing and machine learning: A survey", Pervasive and Mobile Computing 51 (2018) 1-26, was read with interest by the inventors. Furthermore, the paper by van Ee R et al: "Multisensory congruency as a mechanism for attentional control over perceptual selection", J Neurosci (2009) 29: 11641-11649, that related to fundamental neuroscience experiments on the behavioural effects of signal congruency across sensory modalities in our brain was influential to the inventors. It was realised by the inventors that heteromodal congruency could be used as an efficient means for the brain to identify signal relevance in the bombardment of sensory signals in a busy imaging environment, such as CT or MRI. From Degerman A, et al. "Human brain activity associated with audiovisual perception and attention". Neuroimage (2007) 34:1683-1691, it has been shown that there is a machinery of neurons especially devoted to combine attentional functions across sensory modalities, and where these neurons play an important role in attentional control over perception. Thus, it was realised that congruency, then could facilitate multimodal mechanisms of voluntary attentional control, since there is more support for one of the two competing percepts when there is information from another sensory modality that is congruent with it. Indeed experiments revealed that the capacity to attentionally select one of two competing percepts is greatly enhanced when there is such congruency (in some observers over 400% increase in control over perception; see van Ee et al, 2009.

Consequently, the inventors determined that a multimodal signal can be used in a specific embodiment to direct/focus attention to the sedation monitoring stimuli when the signals are presented in rhythmic congruency within a medical imaging environment. This is because it was realised that people are more responsive to multimodal stimuli, and it was realised that this could be used as part of an improved technique to automatically determine the sedated state of a patient in a reproducible and objective manner. Thus, in effect in a detailed specific embodiment a multisensory attention controller was implemented, that uses a combination of signals that vary congruently, i.e. in rhythm over time, with one another. By having a multitude of congruent senses it able to voluntarily direct/focus a patient's brain activity away from the background noise. Consequently, how the signals vary over time can be used to draw the patient's attention and elicit a greater response that from a single form of stimulation. Thus, the way the stimuli vary their waveform, frequency, amplitude and phase, in a congruent manner has been utilized in specific embodiments.

Fig. 5 shows an example of a multi-sensory system as made use of in the presently described apparatus for patient sedation monitoring. Here, use can be made of a person's perception (F), Sensation (G) and stimulation (H) via their senses of vision (A), hearing (B), touch (C), smell (D), and taste (E). Stimulation can be provided in the form of temperature, nudging (virtual touch such as remote nerve and/or muscle stimulation), odour, light, sound and taste (I). Clearly in other examples use can be made of different senses such as balance, pinching (for example to trigger a pain sensation) and further stimuli like a rocking stimuli, and touchless haptic can be made use of.

Thus, the apparatus for patient sedation monitoring overcomes the problem of deciding upon or assessing the sedation level of a patient by providing combinations of at least two of all possible sensory modalities and monitoring the response, which provides for an improvement over that for single sensory modality results, and addresses ambiguity in the outcome previously provided, especially in noisy environments, by generating an objection and reproducible determined sedation state of the patient.

The apparatus for patient sedation monitoring uses multi-modal stimuli generation that combines multiple sensory modality to increase the chance of a response for a given sedated state. Thus, by using multi-modal stimuli at the same time, in a congruent manner, that targets different sensory organs of the human, an increased response is elicited because in effect the "hard-wired" response to stimuli is triggered, that has developed through evolution to enable humans to react to events that are happening. The different stimuli are provided at the same time, and in a similar manner in terms of operating at similar frequencies and this leads to an increased response. Thus, a visual stimuli applied at a frequency of 1Hz applied at the same time as an audible stimuli at a frequency of 1Hz that is combined with haptic touch stimuli applied at 1Hz leads to a much increased response with respect to each of those stimuli being applied separately. Also, when applied at the same time and with the same frequency and/or frequency spectrum and/or same temporal amplitude variation, i.e., congruently, the human's "hard-wired" response is triggered into considering that this is one large stimulus, where evolution could have programmed humans to react to such stimuli, such as could result from a threat, and this leads to an increased response. Further, the combined multi-modality is personalized for the specific person's sensory model. The multi-modal stimuli can be generated from various types of stimulus that targets one or more sensory organs. The different types of stimulus can be selected taking into account the specific attributes of the patient.

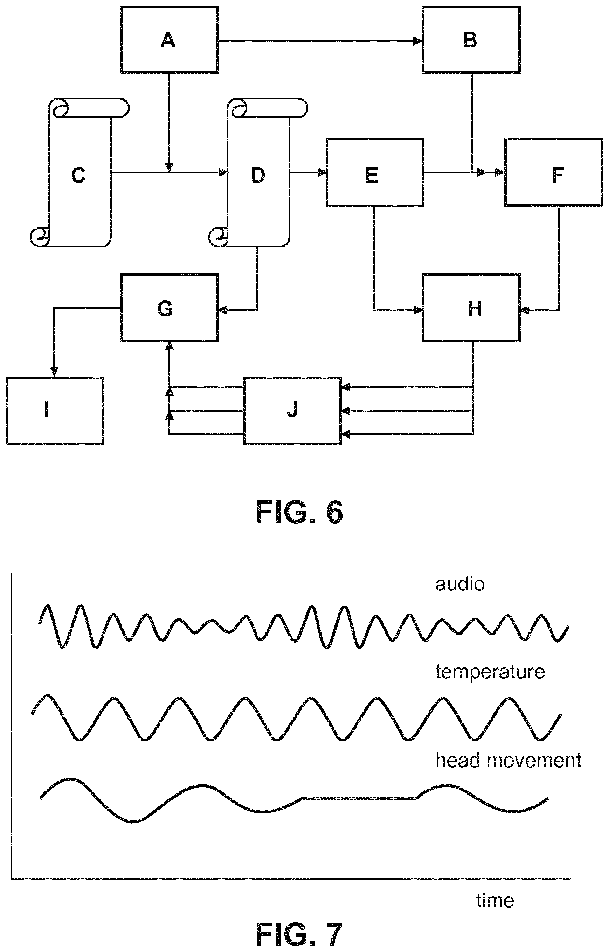

Fig. 6 shows a detailed workflow of operation of the apparatus for patient sedation monitoring being used in a scan environment. The following features and steps are represented as follows:
A: scan type, modality, patient information
B: personal sensory model
C: types of stimulus: touch, sound, light, movement, smell, heat, cold, nerve/muscle stimulation etc
D: selected multimodal stimuli: touch, sound, light
E: congruent multimodal stimuli generator
F: personalization
G: congruent multimodal stimuli assessment
H: stimuli delivery
I: sedation state
J: patient

The workflow shows that from the existing types of stimuli, a subset of stimuli are selected based on scan type, modality, patient information etc. The subset of stimulus are then generated in a temporally congruent manner and in a frequency range that can be around 0.2Hz to 2Hz, or even 0.1Hz to 3Hz. The generated multi-modal stimuli can also optionally be further personalized (as described in embodiment 3 below). The multimodal stimuli can also be delivered autonomously.

Further, since each stimulus chosen for the multi-modal stimuli is known, the corresponding (autonomous) assessment method for determining the sedation state for the applied stimulus is also triggered. Two different assessment methods can then be utilized, however a single method to determine the sedation state can operate on all the responses together. Based upon the congruent multi-sensorial stimuli delivery and associated sedation state assessment, the sedation state is determined and further adaptations, such as the scan sequence order (for example in MR) are adapted.

The following embodiments provide further detail on how the sedation state of a patient can be monitored using the newly developed techniques described here.

### Embodiment 1: Standard sedation state assessment (not optimized for type of stimulus)

In this first embodiment, the multi-modal stimuli is generated and delivered as described above, whilst the assessment of the sedation level can be done manually (by clinical staff) using any known approach.

### Choice of best stimuli to use

In principle the use of any congruent multi-modal stimuli, see the paper by E. Garcia-Ceja et al, will result in better attention focus than either of the individual stimuli applied alone. However, in the environment of an (autonomous) imaging system, there will be certain preferred and less preferred stimuli, and thus the most appropriate two or more stimuli can be selected depending upon the specific situation.

Furthermore, there are certain stimuli whereby the modulation at the preferred frequency range of 0.2Hz to 2 Hz in not easily realized - at least in a repeatable fashion. An example of such a stimulus is a fragrance, where it is known that the olfactory system can become saturated by a fragrance and will only become sensitized again after a period longer than a few seconds and in addition it is difficult to remove a fragrance easily within a few seconds. Thus, fragrance can be utilized, however it is generally not a first choice of stimulus.

For these reasons in a scanning environment it will be preferred to choose from a subset of the following stimuli
- Mechanical haptic stimuli;
- Transcutaneous Electrical Nerve (TENS) stimuli;
- Visual stimuli;
- Taste stimuli;
- Balance organs (rocking the head);
- Audio;
- Nerve and muscle stimulation by using an MRI unit, for example when scanning with such a unit or using a dedicated MR stimulator.

### Multi-modal stimuli generation

Once the preferred multi-modal stimuli are selected, the multi-modal stimuli are generated to get a more reliable and specific stimulus. Examples of stimuli are as follow
Light is auto-focused for e.g. at pupils with right luminance, spectrum etc.;
Sound is generated with the frequency, pitch and loudness to get maximum, sensory response; and
Touch is generated at a sensitive part of skin for stimulation by for example haptic touch.

### Multi-modal stimuli delivery

Thus, to reiterate a congruent, repeatable and objective multi-modal stimuli delivery technique is utilized in order that the response from the sedated patient is elicited. The response or lack of response based on such stimuli provide a more reliable sedation state assessment than that presently achievable.

In a specific example the simultaneous delivery of touchless haptic stimulus at a desired location is provided along with audible stimulus "delivered to a headphone at desired audio quality (pitch, frequency, loudness), and Light is focused light at a particular location, with a required intensity, and modulation frequency, and balance is stimulated via for example the rocking of the head.

In summary, this embodiment, enables a more robust stimulus delivery to assess a given sedation state. As an example, a patient can be provided with a (touchless) haptic stimulus and light at pupil simultaneously and in a temporarily congruent manner, to mimic the tapping of a patient and flashing a light into the patient's eye.

Based on the delivery of multi-modal stimuli, as described above, the response or lack of response is evaluated (which can be manually or autonomously) to decide the current state of sedation for the patient. The response can be manually observed or alternatively autonomously monitored, e.g. by using a camera.

### Embodiment 2: Assessment of sedation state linked with the type of stimuli (generated

### and corresponding assessment)

This embodiment incorporates an autonomous choice of the sedation state assessment method in relation to the choice of stimuli which are to be delivered. For example if the multi-modal stimuli contains touch or light or sound as stimulus, the assessment method will look at the specific response for each type of stimulus. As such the sedation level assessment tool can be chosen. For example:
In this case of a touch stimulus, the response can result in localized muscular movement, which can be assessed by Camera or EMG sensor;
In the case of a light stimulus, the response of the eye can result in eyes blinking or a change in size of the pupil, which can be detected by camera, or MR based sedation assessment or EEG;
In the case of a sound stimulus, the response can be based on a patient tilting their head towards the sound source or ear movement, this can be detected by a camera or movement sensor such as a tilt sensor of accelerometer.

Furthermore if any of the above stimuli is combined with a further stimuli, the method of sensing can still be advantageous.

The response is also correspondingly measured and fed to a sedation state evaluation, as described above.

### Embodiment 3: Taking into account of personal sensory response model

This embodiment is further extended through use of a personalized sensory model response.

Here, sensorial or cognitive limitations of the patient can be taken into account. These can be known, or determined. For example, for a person with hearing disabilities, a sound based stimulus is not suitable and should be removed from the potential list of stimuli. Similarly for a patient with vision impairment, a light based stimulus may not elicit a reliable response and is removed from being one of the multiple stimulus sources. And indeed an elderly patient with cognitive impairment may not respond to questions, and as such this form of stimulation is not selected.

Thus, in one specific example, an assessment is first made of a patient with respect to his or her sensitivity to specific types of stimulus, and also for their values (tolerance, sensitivity etc.), and a personalized sensory response model is created.

### Specific details on a personalised Multi-modal stimuli selection steps:

A patient is exposed to a normalized sensory input from all models, such as sound, light, touch, smell, taste, cold, heat, etc.;
The perceptiveness of each sense is captured to determine the sensitivity of the patient with respect to each type of sensory modal;
The top N senses are selected as a multi-modal sensory input for patient stimuli during sedation level evaluation.
For example in the above steps, after providing all sensory input, it can be found for example that the person is more sensitive to sound, light and (skin) touch. These then can be candidates for the final multi-sensorial stimulus.

Correspondingly, a multi-modal stimuli generator can be adapted to generate the stimuli as per the personalized sensory response model.

In this manner, because all sensory inputs are generated simultaneously, and take into considering the personalized sensory response model of patient, the response from patient is more reliable and objectively measured. This leads to a better determination of sedation state.

### Embodiment 4: Nerve and muscle stimulation by using the MR gradient system

When the imaging system is an MR system, strong currents applied to the MR gradient coils are used to excite sensorial and motor nerves in the patient. The patient feels this as a tickling sensation or spontaneous slight muscle contraction typically at arms or the back. This effect is known as peripheral nerve stimulation (PNS) and conventionally considered to be a side effect of MR imaging, and consequently standard MR scans are designed to widely avoid PNS during MR imaging. However, PNS is not harmful and it has been established that it can be used as one of the multi-modal stimuli as discussed here. Indeed, the response elicited is such that it has been found that such nerve and/or muscle stimulation provided by the MR system alone can be used to determine the sedation state, and need not be combined with other modes of stimulation.

Therefore, typically bipolar trapezoidal gradient waveforms with high current amplitude and pulse durations in the order of 0.1ms to 100ms are applied to the gradient coils. They can be interleaved with waveforms used for MR imaging without compromising MR imaging. The amplitude of the PNS pulses can be varied over time such that the strength of the sensation by the patient varies in sync with the concurrent multi-modal stimuli. PNS can be induced in all patients with state-of-the-art high power gradient systems, but the sensitivity to PNS varies from patient to patient. Therefore, it can be determined that in the multi-modal system, PNS may not be one of the best stimulation modes for specific patients.

### Adaptation of the scan sequence

Given that each sedation state has a corresponding duration, that can be measured based on patient's response, this information enables a specific scan sequence to be automatically adjusted, so that right level of sequence can be prioritized considering the patient's conditions.

Reference was made above to the waveform, frequency, amplitude and phase of the stimuli, and further specific details are now provided

### Waveforms avoiding discontinuities

Concerning the waveform, it was realised that to enhance the response to the multi-modal stimuli the waveform used for the multisensory stimuli do not show discontinuities. This means that to enhance the response the waveform should not have rapid breaks in intensity (for example as exhibited by square wave profiles and saw-tooth profiles) where the intensity changes in an impulsive manner, as this will mask the effect of the periodic variations in trying to focus the attention of patient. Fig. 7 displays three different wave forms, which do not show rapid breaks in intensity. The first wave form produces a sinusoidal variation in signals. Additionally, a zig-zag wave form and a regular series of pulses with a gradual change in intensity (i.e. a part of a sine wave) can be implemented.

### Frequencies and harmonics of waveforms

Optimization for specific patients can be provided, in part, by have an adjustable frequency. The range of available frequencies can be between 3 Hz (fastest) and 1/9 Hz (slowest). The different sensorial signals can vary with the same frequency. However, it is also possible that one or more of the sensorial signals varies with a frequency which is ½, ¼, 1/8..... [more generally 1/(2^n)] of the frequency of another sensorial signal, as this will also assist in focusing the attention of the patient. For example, a repetitive audio signal of a wave breaking with frequency F can be combined with a cushion which rocks the head of the patient from right - to left and back to right (i.e. a single rocking of the head) every 2 times the wave breaks (1/2 F). Furthermore, if the cushion were to temporarily stop the head movement in the upright position, then the rocking of the head of the patient from right - to middle - to left - to middle and back to right (i.e. a single rocking of the head) every 4 times the wave breaks (1/4 F). In these cases, the user received 1 wave sound stimuli with every movement of the head, which they will also associate with the required multisensory stimulation.

### Amplitude variations

The amplitude can also be adjustable so that a various absolute amplitudes and ratios of amplitudes can be imposed. The quantitative value of the amplitude depends on the modality. It is to be noted that changing the amplitude (for fixed frequency) also entails the signal change per second, and consequently it has been found that it is best if the rate of change of the amplitude is slow, however an enhanced response is elicited from the patient even when the amplitude change is fast. For example, for the audio signal of a breaking wave, it is preferred that the amplitude of the signal does not decrease from its maximum value to zero in a single period (F), but that there is at least one period with an amplitude intermediate between the maximum amplitude and zero amplitude inserted into the waveform before the waveform returns to zero.

### Phase difference

Phase differences in stimuli are interesting because the brain is flexible in being able to shift signal interpretation in time. For this reason, it has been found that waveforms can be shifted in phase arbitrarily without leading to any particularly noticeable drop-off in performance.

### An Example of Waveforms according to the invention

Returning to Fig. 7, this displays a set of 3 waveforms, representing an audio, temperature and head movement stimuli (from top to bottom). In this figure, it is seen that the frequency of the audio signal is highest and constant, whilst the amplitude of the signal changes slowly with time. The temperature signal changes at a frequency which is the half of the audio signal, but remains constant with time, whilst that of the head movement changes at a frequency which is one quarter of the audio signal, and at several periods is completely absent (i.e. there are temporarily only 2 stimuli; audio and temperature)

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10) for patient sedation monitoring, comprising:
- a plurality of patient stimulation devices (20);
- at least one sensor device (30); and
- a processing unit (40);
wherein, each patient stimulation device of the plurality of patient stimulation devices is configured to provide a mode of stimulation, and wherein the modes of stimulation for the plurality of patient stimulation devices are different to each other;
wherein, the plurality of patient stimulation devices is configured to provide a multi-modal stimuli to a sedated patient;
wherein, the at least one sensor device is configured to acquire at least one patient response to the multi-modal stimuli; and
wherein, the processing unit is configured to determine a sedation state of the sedated patient comprising utilization of the at least one patient response to the multi-modal stimuli.

2. Apparatus according to claim 1, wherein the plurality of patient stimulation devices are configured to operate in a frequency range of 0.1Hz to 10Hz.

3. Apparatus according to any of claims 1-2, wherein the plurality of patient stimulation devices are configured to provide the modes of stimulation simultaneously to provide the multi-modal stimuli to the sedated patient.

4. Apparatus according to any of claims 1-3, wherein the plurality of patient stimulation devices are configured to provide the modes of stimulation congruently to provide the multi-modal stimuli to the sedated patient.

5. Apparatus according to any of claims 1-4, wherein the plurality of patient stimulation devices are configured to provide stimuli targeted at a plurality of sensory organs of the sedated patient.

6. Apparatus according to claim 5, wherein a first patient stimulation device is configured to target a first sensory organ and a second patient stimulation device is configured to target a second sensory organ.

7. Apparatus according to any of claims 1-6, wherein the processing unit is configured to select the plurality of patient stimulation devices from a larger number of potential patient stimulation devices, the selection comprising utilization of sensory information relating to the sedated patient.

8. Apparatus according to any of claims 1-7, wherein the at least one patient response comprises a plurality of patient responses, wherein the plurality of patient response are different to each other.

9. An image acquisition system (100), comprising:
- an image acquisition unit (110); and
- an apparatus (10) for patient sedation monitoring according to any of claims 1-8;
wherein, the image acquisition unit is configured to acquire image data of the sedated patient; and
wherein, the processing unit is configured to determine at least one scan protocol and/or terminate a scan protocol for the image acquisition unit for the acquisition of the image data comprising utilization of the determined sedation state of the patient.

10. A method (200) for patient sedation monitoring, comprising:
b) providing (210) by a plurality of patient stimulation devices a multi-modal stimuli to a sedated patient, wherein each patient stimulation device of the plurality of patient stimulation devices is configured to provide a mode of stimulation, and wherein the modes of stimulation for the plurality of patient stimulation devices are different to each other
c) acquiring (220) by at least one sensor device at least one patient response to the multi-modal stimuli; and
d) determining (230) a sedation state of the sedated patient comprising utilization of the at least one patient response to the multi-modal stimuli.

11. Method according to claim 10, wherein step d) is carried out by a processing unit, and wherein the method comprises step e) outputting (240) by an output unit the sedation state of the sedated patient.

12. Method according to any of claims 10-11, wherein the method comprises step a) selecting (250) the plurality of patient stimulation devices from a larger number of potential patient stimulation devices, the selection comprising utilizing sensory information relating to the sedated patient.

13. Method according to any of claims 10-12, wherein in step c) the at least one patient response comprises a plurality of patient responses, wherein the plurality of patient response are different to each other.

14. A method (300) of image acquisition comprising:
a1) determining (310) a sedation state of a sedated patient in an image acquisition unit according to the method (200) of any of claims 10-13;
b1) determining (320) by a processing unit at least one scan protocol for the image acquisition unit for the acquisition of image data comprising utilization of the determined sedation state of the patient.

15. A computer program element for controlling an apparatus according to claim any of claims 1-8, which when executed by a processor is configured to carry out the method of any of claims 10-13, and/or for controlling a system according to claim 9, which when executed by a processor is configured to carry out the method of claim 14.
